# EUROPÄISCHE PATENTANMELDUNG

(11) **EP 0 957 171 A2**
(43) Veröffentlichungstag der Anmeldung: **17.11.1999**
(21) Anmeldenummer: 99106669.7
(22) Anmeldetag: 01.04.1999
(51) Int. Cl.: C12N 15/53, C12N 9/04, C12N 1/21, C12Q 1/32

(54) **Rekombinante mikrobielle 3-Hydroxybuttersäure-Dehydrogenase, Verfahren zu deren Herstellung und Verwendung**

(30) Priorität: 08.04.1998 DE 19815685
(71) Anmelder: Roche Diagnostics GmbH, 68298 Mannheim (DE)
(72) Erfinder: Schmuck, Rainer, Dr., 83671 Benediktbeuern (DE); Müller, Rainer, Dr., 82377 Penzberg (DE); Weisser, Harald, Dr., 82347 Bernried (DE); Engel, Alfred, Dr., 82377 Penzberg (DE); Krüger, Kerstin, Dr., 81377 München (DE)

(57) **Zusammenfassung**

DNA-Molekül, umfassend ein Strukturgen, das für ein bakterielles Protein mit der enzymatischen Aktivität von 3-Hydroxybuttersäure-Dehydrogenase (3-HBDH) kodiert, eine das DNA-Molekül beeinhaltende rekombinante DNA, entsprechend transformierte Mikroorganismen, ein Verfahren zur Gewinnung von 3-HBDH durch Züchtung eines geeigneten, transformierten Mikroorganismus sowie ein Verfahren zur Bestimmung von Ketonkörpern in Gegenwart des Enzyms. Bevorzugt ist ein Strukturgen aus Rhodobacter sphaeroides.

SEQUENZPROTOKOLL

<110> Roche Diagnostics GmbH

<120> Rekombinante mikrobielle
      3-Hydroxybuttersäure-Dehydrogenase, Verfahren zu deren
      Herstellung und Verwendung

<130> 4757OAEP

<140> 99106669.7
<141> 1999-04-01

<160> 2

<170> PatentIn Ver. 2.1

<210> 1
<211> 1406
<212> DNA
<213> Rhodobacter species

<400> 1
ccgcccttcg gcgcgtcggg atttgcatat ggcagagaca caaaatccgg tggaagcgcc 60
ggttcgccat acatagctat gccgtgccgg ggttcggcgg ccagcgtcgt gcccagaacg 120
gcaagcccga gagccagcgc ggcgcccctc aggcgggacc ggaattcctg tatcattcgg 180
gcaacagctc ctcggtggcc gatccgcagg gggccagatt tacccgcggg cggtaacctt 240
ttcaaacttt taccttggac agcggcggag aaggtcgtat aagtatccca ctgctcgata 300
ggtttcttgc ctgtatgaaa cctgcctcaa cgacttaacg ccggcttcgc gccggcgttt 360
tttttcgcgc ttcgcgaatg acaggcgccc cggtctccgt gccatctcgc catcccgcgg 420
cccattggct aggtcttggc cagtgcacat gacgggaggc aggatcatgg atctgaacgg 480
caagcgggca atcgtcaccg gctcgaactc gggcatcggg ctgggctgcg ccgaagagct 540
ggcgcgcgcc ggcgccgagg ttgtcatcaa ctccttcacc gaccgcgacg aggatcacgc 600
gctggccgag aagatcggcc gcgaacatgg cgtgagctgc cgctacatcg cggccgacat 660
gtcggacggc gaagcgtgcc gggcgctgat cgagacggcg ggcggctgcg acatcctcgt 720
gaacaacgcg ggcatccagc atgtctcgtc gatcgaggag ttcccggtcg ggaagtggaa 780
cgcgatcctc gcgatcaacc tctcgtcggc cttccacacc acggcggcgg cgctgccggg 840
gatgcgggcg aagggctggg ggcggatcgt caacatcgcc tcggcgcacg ggctgacggc 900
gagcccctac aagtcggcct atgtcgcggc caagcacggg gtggtgggct tcaccaaggt 960
caccgcgctc gagaccgcgg gcaagggcat cacctgcaac gcgatctgcc cgggctatgt 1020
gctgacgccg ctggtcgagg cgcagatccc cgaccagatg aaggcgcacg acatggaccg 1080
cgagacggtg atccgcgagg tgatgctcga ccgccagccc tcgcgccagt tcgcaacgac 1140
cggtcagatc ggcggcacgg tggtgttcct ctgctcgggt gcggccgatc agatcaccgg 1200
gacgacgatc tcggtggatg ggggctggac cgccctctga cgggcggtcc ttcggcaact 1260
cccgggtcag cctctggcga tggcgcgggc gatgtcggtc gcgagccggg cgttcgacag 1320
gacgagcgcc cggttggcct cgagcgaccg gccctcggtc agatcgaaga tcaggtcgag 1380
caggaacggc gtcacggcct tggcgg                                      1406

<210> 2
<211> 257
<212> PRT
<213> Rhodobacter species

<400> 2

## Beschreibung

Die Erfindung betrifft im wesentlichen ein DNA-Molekül, das für ein Protein mit der enzymatischen Aktivität der 3-Hydroxybuttersäure-Dehydrogenase (E.C. 1.1.1.30) kodiert, das DNA-Molekül beinhaltende rekombinante DNA, entsprechend transformierte Mikroorganismen, ein Verfahren zur Gewinnung von 3-Hydroxybuttersäure-Dehydrogenase durch Züchtung eines geeigneten, transformierten Mikroorganismus sowie ein Verfahren zur Bestimmung von Ketonkörpern in Gegenwart des Enzyms.

3-Hydroxybuttersäure-Dehydrogenase (3-HBDH) ist ein Enzym, welches - unter gleichzeitiger Umwandlung von NAD⁺ in NADH - die Oxidation von Hydroxybuttersäure zu Acetessigsäure katalysiert (Bergmeyer, H.U. et al. (1967), Biochem. J. vol. 102, 423-431):

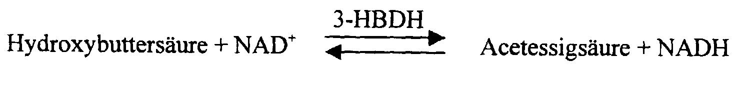

Das Enzym dient zum Nachweis von Ketonkörpern. Unter Ketonkörpern versteht man insbesondere Acetessigsäure, 3-Hydroxybuttersäure und Aceton. Ketonkörper werden bei gesteigerter Lipolyse, z.B. bei Insulinmangel (Diabetes mellitus; TypI-Diabetiker), bei erhöhter Glucagonkonzentration und im Hungerzustand vermehrt gebildet. Die physiologische Konzentration von weniger als 7mg/dl kann dabei aufdas über 10fache ansteigen. Hydroxybuttersäure hat sich über die vergangenen Jahre als ein extrem zuverlässiger Parameter bei der Verfolgung einer Insulin-Therapie erwiesen.

Ketonkörper sind Metaboliten des Fettstoffwechsels. Sie werden in der Leber gebildet und insbesondere anschließend in der Muskulatur metabolisiert. Üblicherweise erfolgt der störungsanfällige qualitative Nachweis der Ketonkörper in Harnproben über Aceton oder Acetessigsäure. 3-Hydroxybuttersäure (3-HB) istjedoch im Vergleich zu Aceton oder Acetessigsäure der dominantere und zuverlässigere Indikator für klinische Diagnosen. Das Verhältnis von 3-HB zu Aceton bzw. Acetessigsäure beträgt im Normalzustand 3:1. Das Verhältnis steigt bei Ketoacidosen auf 6:1 bis 12:1. Mit Hilfe einer geeigneten 3-HBDH sollte es zudem möglich sein, einen quantitativen Test auf 3-Hydroxybuttersäure zu entwickeln.

Es ist bekannt, daß 3-HBDH in einer Reihe eukaryotischer Organismen sowie auch in Prokaryonten vorkommt (Churchill, P. et al. (1992), Biochem. vol. 31, 3793-3799; Marks, A.R. et al. (1992), J. Biol. Chem. vol. 267, 15459-15463; Bergmeyer, H. U. et al. (1967), Biochem. J. vol. 102, 423-431). Insbesondere diverse Bakterien dienen heute als Quelle zur Gewinnung des 3-HBDH-Enzyms. Die klassischen Verfahren zur Herstellung der konventionellen 3-HBDH aus bestimmten Mikroorganismen sind jedoch sehr zeitaufwendig und kostenintensiv, zum einen aufgrund der schwachen Expression von 3-HBDH, zum anderen läßt sich der Fermentationsprozess entsprechender Mikroorganismen nur schlecht reproduzieren.

Die Aufgabe der vorliegenden Erfindung besteht somit in der Bereitstellung eines rekombinanten mikrobiellen Proteins mit der enzymatischen Aktivität der 3-HBDH.

Gelöst wird die Aufgabe durch ein DNA-Molekül,umfassend ein Strukturgen, welches für ein Protein mit der enzymatischen Aktivität der 3-Hydroxybuttersäure-Dehydrogenase kodiert und in einem heterologen Mikroorganismus mittels eines Vektors transformiert und exprimiert wird.

Bei dem erfindungsgemäßen DNA-Molekül bzw. dem 3-HBDH-Strukturgen handelt es sich insbesondere um das für 3-HBDH kodierende Gen, welches aus verschiedenen Mikroorganismen wie beispielsweise aus Bakterien der Gattungen Rhodobacter, Rhodospirillum und Pseudomonas erhältlich ist. Besonders geeignet als Quelle für das erfindungsgemäße DNA-Molekül haben sich Mikroorganismenspecies der Gattung Rhodospirillaceae, insbesondere Rhodobacter sphaeroides erwiesen. Insbesondere wurde ein für 3-HBDH kodierendes Gen aus Rhodobacter sphaeroides (BMTU 109, DSM 12077) welches eine Nukleotidsequenz gemäß SEQ. ID NO. 1 bzw. eine entsprechende aufgrund des genetischen Codes degenerierte Sequenz aufweist, erhalten. Das Rhodobacter-3-HBDH-Strukturgen weist eine Homologie zu entsprechenden eukaryotischen Genen von lediglich 46 bzw. 47% auf und zeichnet sich ferner insbesondere dadurch aus, daß es einen GC-Gehalt von ca. 68 mol% aufweist und das Codon TCG bevorzugt für die Aminosäure Serin (12 von insgesamt 15) sowie CTG/C ausschließlich für die Aminosäure Leucin (insgesamt 16) kodiert. Der Stamm Rhodobacter sphaeroides mit der internen Bezeichnung BMTU 109 ist bei der Deutschen Sammlung von Mikroorganismen und Zellkulturen GmbH (DSM), Mascheroder Weg 1b, D-38124 Braunschweig unter DSM 12077 am 26.03.1998 hinterlegt worden.

Bevorzugt ist erfindungsgemäß weiterhin, wenn die Expression des 3-HBDH-Proteins unter Verwendung einer in SEQ. ID NO. 1 enthaltenen Teilsequenz, insertiert in einen geeigneten Vektor, erfolgt. Als besonders geeignet haben sich erfindungsgemäß solche DNA-Moleküle erwiesen, die einer Nukleotidsequenz von Nukleotid Nr. 467 bis Nr. 1237 der Sequenz gemäß SEQ. ID NO. 1 entsprechen, zu diesen komplementär sind und/oder mit diesen hybridisieren. Solche DNA-Moleküle umfassen beispielsweise Fragmente, Modifikationen, Derivate und alle Varianten der vorstehend beschriebenen DNA-Moleküle. Der Begriff "Hybridisierung" bedeutet in diesem Zusammenhang eine Hybridisierung unter konventionellen Hybridisierungsbedingungen, vorzugsweise unter stringenten Bedingungen (s. z. B. Sambrook et al. (1989) in Molecular Cloning: A Laboratory Manual, Cold Spring Harbor Laboratory, New York, 1.101-1.104 und 9.47-9.55). Insbesondere haben sich erfindungsgemäß folgende stringenten Hybridisierungsbedingungen als geeignet erwiesen: 50% Formamid, 5 x SSC (1 x SSC: 150 mM NaCl, 15 mM Natriumcitrat; pH 7.0), 2% Blockierungsreagenz (Roche Molecular Diagnosties, Kat. No. 1 096 176), 0,1% Lauroylsarcosin und 0.02% SDS sowie eine Temperatur von 42°C.

Ein weiterer Gegenstand der Erfindung ist ein Verfahren zur Herstellung eines Proteins mit 3-HBDH-Aktivität durch Einfügen eines für 3-HBDH kodierenden DNA-Moleküls in einen geeigneten Vektor und Transformation der erhaltenen rekombinanten DNA in einen Mikroorganismus, der die Befähigung aufweist, 3-HBDH im Medium zu produzieren. Geeignete Mikroorganismen für die Expression sind insbesondere solche der Gattung E.coli wie beispielsweise HB101 (ATCC 33694), JM83 (ATCC 35607) sowie RR1 (ATCC 35102).

Erfindungsgemäß haben sich für ein solches Verfahren sämtliche Plasmidvektoren als geeignet erwiesen, die üblicherweise für die Expression in Wirtszellen verwendet werden. Beispielhaft seien hier Vektoren auf Basis des Plasmids pKK177 unter Kontrolle eines T5-Promotors angeführt. Nach Fermentation der transformierten Wirtszellen über mehrere Stunden wird das angereicherte Protein mit der enzymatischen Aktivität der 3-HBDH aus dem Kulturmedium isoliert. Die Isolierung des Enzyms erfolgt im wesentlichen anhand dem Fachmann bekannter Maßnahmen, wie beispielsweise durch Zentrifugation, Aufschluß der Biomasse, Resuspension in geeigneter Pufferlösung, Abtrennung unlöslicher Zellbestandteile und durch verschiedene Chromatographieschritte, beispielsweise mittels hydrophober Säulenmaterialien (z.B. Phenyl-Sepharose) oder/und geeigneter Affinitätschromatographie.

Das rekombinante 3-HBDH-Enzym kann aus dem heterologen Mikroorganismus in guten Ausbeuten mit einer Reinheit von ca. 60% bis 80% sowie einer spezifischen Aktivität von mindestens 200 U/mg erhalten werden. Das erfindungsgemäße rekombinante bakterielle Enzym ist gegenüber den bekannten eukaryotischen 3-HBDH-Enzymen am C-terminalen Ende, bevorzugt um ca. 30 Aminosäuren, verkürzt und weist darüber hinaus keine Phosphatidylcholin-Abhängigkeit auf. Letzteres ist mit dem Vorteil verbunden, daß die Zugabe von externem Lipid (Phosphatidylcholin) zur Entfaltung der Enzymaktivität, d.h. beispielsweise bei der Bestimmung von Ketonkörpern verzichtet werden kann.

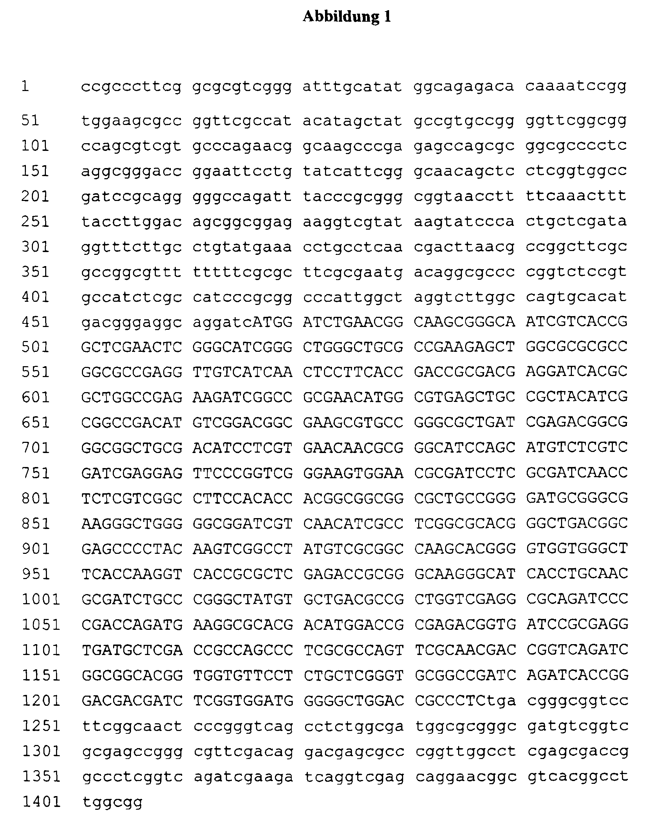

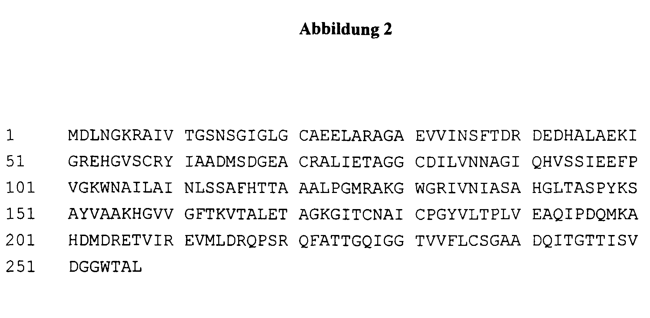

Eine bevorzugte Ausführungsform des erfindungsmäßigen Enzyms ist, wenn eine aus dem Mikroorganismus Rhodobacter sphaeroides erhältliche, für ein 3-HBDH-Protein kodierende DNA-Sequenz in einem E.coli-Stamm, beispielsweise in dem Stamm HB101 (ATCC 33694) exprimiert wird. Das rekombinante mikrobielle 3-HBDH-Enzym konnte so aus dem transformierten Stamm anhand dem Fachmann bekannter Maßnahmen in hoher Ausbeute und mit Reinheit mit einer spezifischen Aktivität von mindestens 200 U/mg Protein erhalten werden. Das Molekulargewicht des aus vier Untereinheiten bestehenden erfindungsgemäßen Enzyms liegt zwischen 24000 und 30000 Dalton (SDS-PAGE). Ferner weist eine bevorzugte Ausführungsform des Enzyms einen PI von 5,90, ein pH-Optimum von ca. pH 7 bis 8,5 (0,2 M Kaliumphosphat bzw. Tris/HCl-Puffer) sowie eine maximale Enzymaktivität bei ungefähr 50°C aufund zeichnet sich durch die in SEQ. ID NO. 2 bzw. Abbildung 2 angegebene Aminosäuresequenz aus.

Ein weiterer Gegenstand der Erfindung ist ein Verfahren zur Bestimmung von Ketonkörpern in Gegenwart des erfindungsgemäßen rekombinanten mikrobiellen Enzyms. Der Test kann prinzipiell, d.h. bezüglich der sonstigen benötigten Komponenten, nach bekannten Maßnahmen erfolgen (z.B. Bergmeyer, H.U. et al. (1967), Biochem. J. vol. 102, 423-431).

### Legende zu den Abbildungen:

Abbildung 1:
   Nukleotidsequenz der 3-HBDH aus Rhodobacter spec. (Nukleotide 467-1237).
Abbildung 2:
   Aminosäuresequenz der 3-HBDH aus Rhodobacter spec.
Abbildung 3:
   SDS-PAGE einer hoch-angereicherten 3-HBDH aus Rhodobacter spec. Das Protein wurde in E.coli exprimiert und mittels Phenyl-Sepharose-Chromatographie gereinigt. Es wurden Proben mit 1 µg und 0,5 µg Protein aufgetragen.
Abbildung 4:
   Kinetische Daten (Absorption/Zeit) für die Bildung von Acetat und NADH mit dem erfindungsgemäßen Enzym gemäß Beispiel 2;
   Reihe 1, Reihe 2 (Tabelle 2).

Die folgenden Beispiele erläutern die Erfindung weiter:

### Beispiel 1:

### Klonierung der 3-HBDH

Zur Isolierung der chromosomalen DNA wurden 0.7 g Bakterien-Naßgewicht (Rhodobacter sphaeroides) in 2 ml 50 mM Tris pH 8.0, 1 mM EDTA, 6.7% Saccharose resuspendiert und für 15 Min. bei 37°C inkubiert. Anschließend wurden 50 mg Lysozym zugegeben und weitere 10 Min. bei 37°C inkubiert. Um DNAsen zu inhibieren, wurden 1.25 ml 0.5 M EDTA pH 8.0 zugegeben. Die Bakterien wurden durch die Zugabe von 0.75 ml 20% SDS, 50 mM Tris pH 8.0 20 mM EDTA für 10 Min. bei 37°C lysiert. Anschließend wurden 2 mg Proteinase K zugegeben und bei 37°C für 3 Std. inkubiert. 5 M Natriumperchlorat wurde bis zu einer Endkonzentration von 1 M zugegeben. Die Lösung wurde nun zweimal mit Phenol/Chloroform/Isoamyalkohol (25:24:1) und zweimal mit Chloroform extrahiert. Die chromosomale DNA wurde mit dem 2.5fachen Volumen 100% Ethanol und 1/10 Volumen 3 M Natriumacetat für 30 Min. bei -20°C gefällt. Das Pellet wurde mit 5 ml 70% Ethanol gewaschen, luftgetrocknet und in TE (10 mM Tris/HCl pH 7,8; 1 mM EDTA) resuspendiert. Um RNA zu verdauen, wurde die Lösung mit 100 µg/ml RNaseA für 2 Std. bei 37°C inkubiert.

Alternativ kann die Isolierung chromosomaler DNA mit Hilfe des von der Firma QIAGEN vertriebenen Genomic DNA Purification Kits nach den Angaben des Herstellers erfolgen.

Das für die N-terminalen Aminosäuren 1-163 (SEQ. ID NO. 2) kodierende DNA-Fragment wurde mittels PCR unter der Verwendung degenerierter Primer, die von den vorliegenden Peptidsequenzen abgeleitet wurden, amplifiziert. Das DNA-Fragment wurde in einen geeigneten Vektor, z.B. den Vektor pCRII (Clark, J.M. (1988), Nucl. Acids Res. vol. 16, 9677-9686 und Mead, D. et al. (1991), Bio/Technology vol. 9, 657-663) kloniert und sequenziert. Als hilfreich hat sich hierbei der von der Firma Invitrogen angebotene TA Cloning Kit erwiesen.

Die Isolierung des 3'-terminalen Endes des 3-HBDH-Gens (entspricht Aminosäureresten 164-257, SEQ. ID NO. 2) erfolgte über Southern Blot Analyse und der Herstellung einer partiellen Genbank. Je 10 µg chromosomaler DNA wurden mit verschiedenen Restriktionsenzymen (*Bam* HI, *Nae* I, *Sma* I, *Pst* I, *Nco* I) verdaut, gelelektrophoretisch aufgetrennt und aufeine Nylon-membran transferiert. Als Sonde zur Detektion homologer Fragmente in der chromosomalen DNA diente ein 514 bp Eco-RI-Fragment, das die ersten 489 bp des 3-HBDH Gens enthielt. Die Markierung dieses Fragmentes erfolgte durch Random Priming gemäß Maniatis et al., Molecular Cloning, A Laboratory Manual, 2nd. Edition, Cold Spring Harbor Laboratory Press (1989) mit Digoxigenin-dUTP (DIG High Prime DNA Labeling and Detection Starter Kit II, Fa. Boehringer Mannheim). DNA-Restriktionsfragmente, die ein positives Signal mit der Sonde ergaben, wurden aus dem Gel nach Maniatis et al. (1989), Molecular Cloning, A Laboratory Manual, 2nd. Edition, Cold Spring Harbor Laboratory Press) extrahiert und zur Herstellung einer partiellen Genbank in den Vektor pBluescript II SK+ (Fa. Stratagene) ligiert und in E.coli XL1 Blue transformiert.

Positive Klone wurden mit einer Sonde im Colony lift detektiert, die die 5'-terminalen 152 bp des 3-HBDH-Gens enthielt. Die Sonde wurde mit Hilfe der PCR synthetisiert und ebenfalls durch Random Priming mit Digoxigenin-dUTP markiert.

1% der Klone, die *Bam* HI-Restriktionsfragmente enthielten, zeigten ein positives Signal mit der verwendeten Sonde. Zwei ausgewählte *Bam* HI-Klone wurden mit verschiedenen internen Primern sequenziert (SEQ. ID NO. 1). Die aus der Sequenz abgeleitete Aminosäuresequenz entspricht der gemäß SEQ. ID NO. 2 widergegebenen Sequenz.

Zur Klonierung des 3-HBDH-Gens in die Expressionsvektoren pKK177-HB und pKKT5 wurde das 3-HBDH-Gen über die PCR mit der Taq-Polymerase (Fa. Boehringer Mannheim) amplifiziert. Die verwendeten Oligonukleotide dienten gleichzeitig der Einführung von Restriktionsenzymschnittstellen (*Eco* RI am 5'-Ende und *Hin* dIII am 3'-Ende des 3-HBDH-Gens). Die PCR-Zyklen waren: 3 Minuten 95°C (Denaturierung); 30 Zyklen: 2 Minuten 95°C (Denaturierung), 1 Minute 57°C (Annealing), 1 Minute 72°C (Polymerisation); 7 Minuten 72°C (Polymerisation). Als Template-DNA dienten die *Bam* HI-Klone, die im Colony Lift ein positives Signal ergeben hatten und sequenziert waren. Das amplifizierte DNA-Fragment wurde nach Vogelstein, B. und Gillespie, D. (1979), Proc. Natl. Acad. Sci. USA vol. 76, 615-619 beispielsweise mit Hilfe des High Pure PCR Purification Kits (Fa. Boehringer Mannheim) gereinigt, mit den Restriktionsenzymen *Eco* RI und *Hin* dIII geschnitten und nach Auftrennung aufeinem 1%igen Agarosegel aus dem Gel eluiert, in die beiden Expressionsvektoren ligiert und in E.coli XL1 Blue damit transformiert. Positive Klone wurden durch Restriktionsverdau mit den gleichen Enzymen identifiziert und zur Kontrolle sequenziert.

Verschiedene E.coli Stämme (z.B. RR1 (ATCC 35102), HB101 (ATCC 33694), JM83 (ATCC 35607) wurden mit dem rekombinanten Plasmid 3-HBDH x pKKT5 zusammen mit dem Helferplasmid pUBS520 transformiert.

Zur Expression der 3-HBDH wurden Übernachtkulturen der E.coli Stämme 1/60 in Luria-Broth (mit 100 µg/ml Ampicillin und 50 µg/ml Kanamycin) überimpft und bei einer OD550 von 0,5 mit IPTG (Isopropylthiogalactosid; 1 mM Endkonzentration) induziert. Nach der Induktion wurden die Kulturen noch weitere 4 Stunden bei 37°C inkubiert. Die Kulturen wurden abzentrifugiert, das Zellpellet in 50 mM Kaliumphosphat, pH 7,6, 0,1 mM Calciumchlorid, 0,1 mM Magnesiumchlorid resuspendiert und mit Hilfe der French Press aufgeschlossen. Nach Filtration des Aufschlusses wurde der Extrakt mit Ammoniumsulfat versetzt bis eine Leitfähigkeit von 150 mS/cm erreicht wurde. Nach Justierung des pH-Werts auf 7,6, wurde das Filtrat aufeine Phenyl-Sepharose Säule gegeben und mit einem linearen Gradienten, der von 1,2 M bis 0 M Ammoniumsulfat reichte, fraktioniert. Fraktionen, die 3-HBDH-Aktivität enthielten, wurden gegen 0,05 M Tris-HCI, pH 7,8, 1 mM Dithiothreitol dialysiert.

Es wurde aufdiese Weise ein Protein mit 3-HBDH-Aktivität in einer Reinheit von ca. 80% und einer spezifischen Aktivität von über 200 U/mg bis ca. 250 U/mg Protein erhalten (1 Unit Enzymaktivität entspricht der Menge an Enzym, die die Umsetzung von 1 µmol Substrat pro Minute katalysiert). Tabelle 1 gibt das Ergebnis eines Reinigungsverfahrens für das erfindungsgemäße Enzym wider.

**Tabelle 1**

| Probe/Schritt | %-Rest-Akt. | MU | SA |
|---|---|---|---|
| Ausgang in Trübe | 100 | 12,6 | - |
| n. Zentri. inkl. Polymin G20 | 101 | 12,7 | 170 |
| + AS und Zentri. | 100 | 12,6 | 140 |
| Phenyl-Sepharose | 84 | 10,6 | 230 |
| n. Ultrafiltration | 78 | 9,87 | 240 |
| n. Verschnitt und Sterilfilt. | 83 | 10,5 | 220 |
| n. Lyophilisation (Raffinose) | 62 | 7,81 | >225 |

### Beispiel 2:

Der enzymatische Test wurde wie folgt durchgeführt. Eine Pufferlösung (1 ml 0,2 M Tris-HCI, pH 8,0), 0,1 ml NAD⁺ (20 mg/ml wässrige Lösung) und 0,25 ml einer Substratlösung (Natrium-β-Hydroxybutyrat, 20 mg/ml wässrige Lösung) wurden bei 25°C konditioniert (in einer Küvette mit einem 1 cm Lichtweg). Die Reaktion wurde durch Zugabe von 0,025 ml verdünnter Lösung enthaltend des erfindungsgemäßen Enzyms gestartet und die Reaktionsgeschwindigkeit durch Messung der linearen Änderung der Absorption bei 340 nm verfolgt (Tabelle 2).

**Tabelle 2**

| Zeit [mm] | Reihe 1 Absorption bei 340 nm | Reihe 2 Absorption bei 340 nm |
|---|---|---|
| 0 | 0,1693 | 0,1624 |
| 0,5 | 0,18 | 0,1777 |
| 1 | 0,1927 | 0,1869 |
| 1,5 | 0,2057 | 0,1997 |
| 2 | 0,2249 | 0,2146 |
| 2,5 | 0,2309 | 0,224 |
| 3 | 0,2436 | 0,2376 |
| 3,5 | 0,2561 | 0,2499 |
| 4 | 0,2691 | 0,2623 |
| 4,5 | 0,2827 | 0,2757 |
| 5 | 0,2951 | 0,2883 |
| 5,5 | 0,3077 | 0,3001 |
| 6 | 0,3203 | 0,3134 |
| 6,5 | 0,3333 | 0,326 |
| 7 | 0,3466 | 0,339 |
| 7,5 | 0,3591 | 0,3513 |
| 8 | 0,3717 | 0,3641 |
| 8,5 | 0,3843 | 0,3766 |
| 9 | 0,3973 | 0,3891 |
| 9,5 | 0,4099 | 0,4091 |
| 10 | 0,4227 | 0,4139 |
| | | |
| Rate | 0,0254 | 0,0251 |
| Standardabweichung | 0,0015 | 0,009 |
| Ergebnis | 0,2541 | |

Abbildung 4 zeigt die entsprechende graphische Auswertung.

## Patentansprüche

1. Isoliertes DNA-Molekül, umfassend ein Strukturgen, das für ein Protein mit der enzymatischen Aktivität von 3-Hydroxybuttersäure-Dehydrogenase (E.C. 1.1.1.30) kodiert und aus einem Mikroorganismus erhältlich ist.

2. DNA-Molekül gemäß Anspruch 1, dadurch gekennzeichnet, daß das Strukturgen aus Bakterien der Gattung Rhodobacter erhältlich ist.

3. DNA-Molekül gemäß Anspruch 1 oder 2, dadurch gekennzeichnet, daß das Strukturgen aus Rhodobacter sphaeroides (BMTU 109, DSM 12077) erhältlich ist.

4. DNA-Molekül nach einem der Ansprüche 1 bis 3, dadurch gekennzeichnet, daß das Strukturgen für ein Protein mit einer Aminosäuresequenz gemäß SEQ. ID NO. 2 bzw. Abbildung 2 kodiert.

5. DNA-Molekül nach einem der Ansprüche 1 bis 4, daß es ein Strukturgen gemäß der Sequenz SEQ. ID NO. 1 bzw. Abbildung 1 enthält.

6. Rekombinante DNA, die fähig ist, in einem Mikroorganismus repliziert zu werden, umfassend einen Plasmidvektor und ein DNA-Molekül nach einem der Ansprüche 1 bis 5.

7. Rekombinante DNA nach Anspruch 6, worin der Plasmidvektor pKK177 oder ein davon abgeleitetes Plasmid ist.

8. Mikroorganismus transformiert mit einer rekombinanten DNA nach einem der Ansprüche 6 oder 7.

9. Mikroorganismus nach Anspruch 8, dadurch gekennzeichnet, daß es sich um E.coli handelt.

10. Verfahren zur Herstellung eines Proteins mit der enzymatischen Aktivität von 3-Hydroxybuttersäure-Dehydrogenase, umfassend die Schritte der (a) Transformation eines Mikroorganismus mit einer rekombinanten DNA nach Anspruch 6 oder 7, (b) Kultivierung des transformierten Mikroorganismus, so daß sich besagtes Protein in dem Kulturmedium anreichert und (c) Gewinnung des Proteins aus dem Kulturmedium.

11. Isoliertes Protein mit der enzymatischen Aktivität von 3-Hydroxybuttersäure-Dehydrogenase erhältlich nach dem Verfahren gemäß Anspruch 10.

12. Bakterielles Protein gemäß Anspruch 11, dadurch gekennzeichnet, daß es eine Aminosäuresequenz gemäß SEQ. ID NO. 2 bzw. Abbildung 2 aufweist.

13. Verfahren zur enzymatischen Bestimmung von Ketonkörpern in einer biologischen Probe in Gegenwart eines Proteins mit 3-Hydroxybuttersäure-Dehydrogenase-Aktivität und eines Coenzyms in reduzierter oder oxidierter Form, dadurch gekennzeichnet, daß ein Protein gemäß der Ansprüche 11 oder 12 verwendet wird und der Verbrauch bzw. die Bildung an Coenzym optisch bestimmt wird.
